Europäisches Patentamt

European Patent Office

Office européen des brevets

(11) Veröffentlichungsnummer: **0 450 330 A1**

(12) **EUROPÄISCHE PATENTANMELDUNG**

(21) Anmeldenummer: **91103355.3**

(22) Anmeldetag: **06.03.91**

(51) Int. Cl.⁵: **A61M 39/00**, F16L 33/00, B29C 65/54

(30) Priorität: **31.03.90 DE 9003765 U**

(43) Veröffentlichungstag der Anmeldung:
**09.10.91 Patentblatt 91/41**

(84) Benannte Vertragsstaaten:
**AT BE CH DE DK ES FR GB IT LI LU NL SE**

(71) Anmelder: **B. Braun Melsungen AG**
**Carl-Braun Strasse**
**W-3508 Melsungen(DE)**

(72) Erfinder: **Bubelach, Lutz-Günther**
**Parkstrasse 12**
**W-1000 Berlin 42(DE)**

(74) Vertreter: **Selting, Günther, Dipl.-Ing. et al**
**Patentanwälte von Kreisler, Selting, Werner**
**Deichmannhaus am Hauptbahnhof**
**W-5000 Köln 1(DE)**

(54) **Ansatzstück mit einem Schlauch.**

(57) Bei dem Ansatzstück mit einem Schlauch (10), weist der in eine Aufnahmebohrung (26) des Ansatzstückkörpers (20) eingesetzte Schlauch (10) einen angeformten Endflansch (11) auf, der gegen einen Anschlag in der Aufnahmebohrung (26) anliegt. Ein Ringspalt zwischen dem Außenumfang des Schlauches (10) und der Innenfläche der Aufnahmebohrung (26) ist mit Klebstoff (27) ausgefüllt. Dabei liegt gegen den als Ringschulter (17) am inneren Ende der Aufnahmebohrung (26) ausgebildeten Anschlag die stirnseitige Fläche (12) des Endflansches (11) des Schlauches (10) an. Auf diese Weise gelingt es, einen Schlauch aus chemisch inertem Material durch Klebstoff gegen Axialverschiebung in beiden Richtungen in der Aufnahmebohrung des vorgefertigten Ansatzstückkörpers zu sichern.

FIG.2

EP 0 450 330 A1

Die Erfindung bezieht sich auf ein Ansatzstück mit einem Schlauch, bei dem der in eine Aufnahmebohrung des Ansatzstückkörpers eingesetzte Schlauch einen angeformten Endflansch aufweist, der gegen einen Anschlag in der Aufnahmebohrung anliegt und bei dem ein Ringspalt zwischen dem Außenumfang des Schlauches und der Innenfläche der Aufnahmebohrung mit Klebstoff ausgefüllt ist.

In der Medizin müssen für bestimmte Medikamentenkombinationen Schläuche aus Polyethylen benutzt werden, weil diese Medikamente sich bei Schläuchen aus Polyvinylchlorid mit dem Weichmacher des PVC verbinden und dabei ihre Wirkung verlieren und/oder unerwünschte Nebeneffekte im Patientenkörper hervorrufen. Da der Kunststoff Polyethylen chemisch inert ist, gelingt es nicht zur Verbilligung der Herstellung, Schläuche aus Polyethylen mit einem vorgefertigten Ansatzstückkörper aus anderem Kunststoff durch Klebung zu verbinden. Um Polyethylenschläuche mit dem Ansatzstückkörper zu versehen, ist es deshalb erforderlich, den Schlauch zu umspritzen. Zu diesem Zweck muß der Schlauch in eine Spritzgießform eingelegt werden und der Ansatzstückkörper wird nach Schließen der Form um den Schlauch gespritzt. Es erfolgt eine thermische Verbindung. Dieses Vorgehen ist sehr kostenaufwendig und die Stückzahl wird von der Maschinenleistung abhängig gemacht.

Zur Ermöglichung der Befestigung eines Kunststoffschlauches an einem Ansatzstückkörper durch Verklebung ist die aus US-A-3 851 647 bekannte, eingangs erwähnte Anordnung eines Ansatzstückes mit einem Schlauch bekannt. Dabei ist der Anschlag eine einwärts gerichtete Ringwulst innerhalb der Aufnahmebohrung, gegen deren eine Seite die schlauchseitige Ringfläche des Endflansches des Schlauches anliegt und an dessen andere Seite sich der Klebstoff anschließt, der den Ringspalt zwischen dem Außenumfang des Schlauches und der Innenfläche der Aufnahmebohrung ausfüllt. Die Wahl des Kunststoffes für den Schlauch ermöglicht in diesem Falle die Haftung des Klebstoffes sowohl an der Innenfläche der Aufnahmebohrung als auch an dem Schlauch. Wenn jedoch der Schlauch aus Polyethylen besteht und mit dem Klebstoff keine Klebverbindung eingeht, bietet die bekannte Anordnung nur einen Schutz gegen Herausziehen des Schlauches nach vorne und verhindert nicht seine ungehinderte Verschiebung nach hinten. Der Klebstoff würde dabei nur eine in der Aufnahmebohrung festsitzende Passungsmanschette bilden, die den Ringspalt um das Schlauchende ausfüllt, jedoch dieses nicht in der Aufnahmebohrung unbeweglich festhält. Die Ringwulst hat bei dem bekannten Ansatzstück mit Schlauch allein die Aufgabe, beim Einführen des Schlauches in die Aufnahmebohrung seine Endposition in dieser zu definieren, damit das in der Aufnahmebohrung steckende, zu verklebende Schlauchstückende nicht zu kurz ist. Für die Halterung eines Schlauches aus einem Material, das an Klebstoff nicht haftet, z.B. Polyethylen, ist die Ringwulst bedeutungslos.

Der Erfindung liegt die Aufgabe zugrunde, das bekannte Ansatzstück mit einem Schlauch so zu verbessern, daß ein Schlauch aus chemisch inertem Material durch Klebstoff gegen Axialverschiebung in beiden Richtungen in der Aufnahmebohrung des vorgefertigten Ansatzstückkörpers gesichert ist.

Diese Aufgabe wird erfindungsgemäß dadurch gelöst, daß gegen den als Ringschulter am inneren Ende der Aufnahmebohrung ausgebildeten Anschlag die stirnseitige Fläche des Endflansches des Schlauches anliegt.

Der Klebstoff, der nur an der Innenfläche der Aufnahmebohrung des als Kunststoff-Spritzgußteil vorgefertigten Ansatzstückkörpers und nicht an dem Schlauch, z.B. aus Polyethylen, haftet, bildet einen festsitzenden, hülsenförmigen Füllkörper in der Aufnahmebohrung, der den Schlauch in der Aufnahmebohrung verankert. Da der Endflansch des Schlauches zwischen der Ringschulter und der Klebstoffüllung des Ringspaltes fest eingeschlossen ist, wird das Schlauchende in Axialrichtung völlig unbeweglich gehalten und es ergibt sich eine sichere Verbindung zwischen nicht klebfähigem Schlauch und Ansatzstück. Die Erfindung ermöglicht daher die preisgünstige Herstellung von Ansatzstücken mit Schlauch beliebiger Zweckbestimmung zur medizinischen Verwendung.

Zur Anbringung des Endflansches an einem Polyethylenschlauch wird mit Wärme an den Rand seines Endes ein umlaufender Ring angeschmolzen, der zueinander parallele, zur Bohrungslängsachse senkrechte Ringflächen hat, von denen die dem Schlauch zugewandte Ringfläche nach der Art einer Hinterschneidung mit der Klebstoffüllung zusammenwirkt und die gegenüberliegende Ringfläche auf der Ringschulter der Aufnahmebohrung sitzt. Vorteilhaft ist es, wenn der Durchmesser der Aufnahmebohrung im Bereich der ebenfalls zur Bohrungslängsachse senkrechten Ringschulter dem Außendurchmesser des Endflansches im wesentlichen gleich ist, damit der Endflansch bei Anlage gegen die Ringschulter passend in der Aufnahmebohrung steckt und der Klebstoff nicht in haarfeine Spalte zwischen dem Außenumfang des Endflansches und der Innenfläche der Aufnahmebohrung gepreßt werden muß, um jegliches Spiel an dem Endflansch auszuschließen. Vorzugsweise erweitert sich die Aufnahmebohrung gegen ihr äußeres Ende konisch. Hierdurch wird das Einschieben des Endflansches in die Aufnahmebohrung bis zur Anlage gegen die Ringschulter erleichtert und auch das Einbringen des Klebstoffes, der vorzugsweise den Ringspalt von der schlauchseitigen Fläche des Endflansches bis zum äußeren Ende der Aufnahmebohrung ausfüllt, vereinfacht. Nach Aushärten des Klebstoffes, der eine Verbindung mit dem als Spritzgießteil hergestellten Ansatzstückkörper eingeht, ist der Polyethylenschlauch dicht und reißfest mit dem Ansatzstückkörper verbunden.

Am äußeren Ende der Aufnahmebohrung ist eine konische Senkung vorgesehen und der Klebstoff schließt mit der Außenfläche des Ansatzstückkörpers etwa bündig ab. Das Fehlen von Klebstoffüberstand über die Aufnahmebohrung ist aus ästhetischer Sicht günstig.

Da Polyethylen sich in der Medizin als bevorzugter Kunststoff für Schlauchleitungen erwiesen hat, besteht der Schlauch vorzugsweise aus Polyethylen, während der Ansatzstückkörper ein Spritzgießteil aus anderem Kunststoff ist und der Klebstoff aus der Gruppe der Methacrylat/Acrylat-Harze gewählt ist. Methacrylat/Acrylat-Harze härten unter UV-Bestrahlung in Sekundenschnelle aus und sind deshalb besonders günstig. Die Wahl des Klebers bestimmt die Zeitdauer der Aushärtung und somit den Produktionsrhythmus bei der Montage von Ansatzstücken mit Schläuchen.

In der Zeichnung ist ein Ausführungsbeispiel der Erfindung schematisch dargestellt.

Fig. 1 zeigt ein Ansatzstück in Form einer verriegelbaren Schlauchkupplung, an dessen Körper ein Schlauch beliebiger Länge befestigt ist, in Draufsicht, und

Fig. 2 veranschaulicht im Längsschnitt die Verbindung zwischen Schlauch und Ansatzstückkörper in vergrößertem Maßstab.

Ein Schlauch 10 aus Polyethylen mit kreisförmigem Querschnitt ist an seinem äußeren Ende mit einem durch Wärmeeinfluß angeschmolzenen Endflansch 11 versehen, der flache Ringform mit kreiszylindrischem Außenumfang hat. Die stirnseitige Fläche 12 des Endflansches 11 ist eben und verläuft parallel zu der schlauchseitigen Ringfläche 13 des Endflansches 11. Die Flächen 12 und 13 sind jeweils senkrecht zur Längsachse des Schlauches 10 ausgerichtet. Am Übergang der Fläche 13 zum Ringumfang 14 des Endflansches 11 befindet sich eine leichte konvexe Abrundung 15. Bei dem gezeichneten Beispiel entspricht die radiale Breite des über den Außenumfang des Schlauches 11 vorstehenden Teiles des Endflansches etwa der Wanddicke des Schlauches 10.

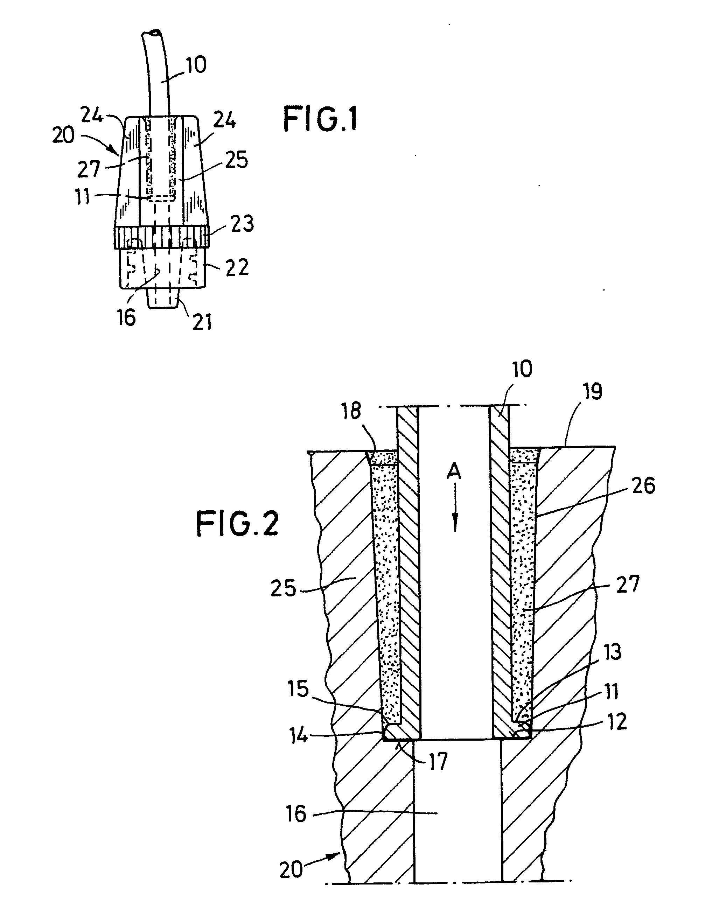

Der Ansatzstückkörper 20 ist ein Spritzgießteil vorzugsweise aus Styrolux (Styrol-Butadien-Blockcopolymere) oder ABS (Acrylmitril-Butadien-Styrol-Copolymere) glasklarer Beschaffenheit. Bis auf die Notwendigkeit des Vorhandenseins eines durchgehenden axialen Kanals 16 ist sein äußerer Aufbau und seine Bestimmung für die Erfindung nicht wesentlich. Im vorliegenden Falle handelt es sich um eine Schlauchkupplung mit einem koaxialen Außenkonus 21, der über einen Teil seiner Länge von einer zylindrischen Mantelhülse 22 umgeben ist, auf deren Innenseite sich ein Gewinde befindet. An einen Rändelring 23 schließt sich koaxial ein Griffteil mit radialen Flügeln 24 an, die von einem Röhrenabschnitt 25 ausgehen und so lang wie dieser sind. In dem Röhrenabschnitt 25 und dem Außenkonus 21 erstreckt sich der Kanal 16, der in dem Röhrenabschnitt 25 einen Teil größeren Durchmessers aufweist, welcher eine im Querschnitt kreisförmige Aufnahmebohrung 26 für das Ende des Schlauches 10 bildet. Die Aufnahmebohrung 26 ist etwas kürzer als der Röhrenabschnitt 25 und endet innen an einer ebenen Ringschulter 17, die zu der Längsachse des Kanals 16 konzentrisch und senkrecht orientiert ist. Wie Fig. 2 zeigt, sind die Abmessungen so getroffen, daß die stirnseitige Fläche 12 des Endflansches 11 des Schlauches 10, die gegen die Ringschulter 17 anliegt, radial breiter als die Ringschulter 17 ist, so daß der Schlauch 10 in den Kanal 16 radial etwas vorspringt.

Dies ist unerheblich, wenn Flüssigkeit in Richtung des Pfeils A strömt. Bei entgegengesetzter Strömungsrichtung oder bei Ausbildung des Ansatzstückkörpers 20 als Katheteransatz bei einem Punktionsbesteck ist es zur Vermeidung von Wirbelbildungen oder des Anstoßens der Punktionskanülenspitze gegen die stirnseitige Fläche 12 des Endflansches 11 günstiger, die freien Querschnitte des Kanals 16 in dem Ansatzstückkörper 20 und in dem Schlauch 10 einander anzupassen.

Die Aufnahmebohrung 26 erweitert sich von der Ringschulter 17 nach außen leicht konisch. Ihr Durchmesser im Bereich der Ringschulter 17 ist etwa gleich dem Außendurchmesser des Endflansches 11. Am entgegengesetzten Ende der Aufnahmebohrung 26 befindet sich eine konische Senkung 18. Die glatte oder aufgerauhte Innenfläche der Aufnahmebohrung 26 bildet mit dem in diese eingesetzten kreiszylindrischen Außenumfang des Schlauches 10 einen Ringspalt, der mit Klebstoff 27, vorzugsweise einem Epoxid-Kleber, ausgefüllt ist. Der Klebstoff 27 erstreckt sich von der schlauchseitigen Ringfläche 13 des Endflansches 11 bis zu der Außenfläche 19 des Ansatzstückkörpers 20 und schließt mit dieser bündig ab. Der Klebstoff 27 bildet einen auf dem Innenumfang kreiszylindrischen und auf dem Außenumfang leicht konischen Hülsenteil, der mit seiner Außenfläche eine fest haftende Verbindung mit dem als Spritzgießteil gefertigten Ansatzstück 20 eingeht. Die festsitzende Klebstoffüllung 27 bildet einen dem Endflansch 11 des Schlauches 10 vorgelagerten Riegel, der ein Herausziehen des Schlauches 10 aus der Aufnahmebohrung 26 nach vorne verhindert. Ein Hineinschieben des Schlauches 10 in den Kanal 16 nach hinten wird durch die Ringschulter 17 blockiert.

## Patentansprüche

1. Ansatzstück mit einem Schlauch (10), bei dem der in eine Aufnahmebohrung (26) des Ansatzstückkörpers (20) eingesetzte Schlauch (10) einen angeformten Endflansch (11) aufweist, der gegen einen Anschlag in der Aufnahmebohrung (26) anliegt, und bei dem ein Ringspalt zwischen dem Außenumfang des Schlauches (10) und der Innenfläche der Aufnahmebohrung (26) mit Klebstoff (27) ausgefüllt ist, **dadurch gekennzeichnet,** daß gegen den als Ringschulter (17) am inneren Ende der Aufnahmebohrung (26) ausgebildeten Anschlag die stirnseitige Fläche (12) des Endflansches (11) des Schlauches (10) anliegt.

2. Ansatzstück nach Anspruch 1, dadurch gekennzeichnet, daß der Durchmesser der Aufnahmebohrung (26) im Bereich der Ringschulter (17) dem Außendurchmesser des Endflansches (11) im wesentlichen gleich ist, daß die Aufnahmebohrung (26) sich gegen ihr äußeres Ende konisch erweitert und daß der Klebstoff (27) den Ringspalt in der Aufnahmebohrung (26) von der schlauchseitigen Fläche (13) des Endflansches (11) bis zum äußeren Ende der Aufnahmebohrung (26) ausfüllt.

3. Ansatzstück nach Anspruch 1 oder 2, dadurch gekennzeichnet, daß am äußeren Ende der Aufnahmebohrung (26) eine konische Senkung (18) vorgesehen ist und daß der Klebstoff (27) mit der Außenfläche (19) des Ansatzstückkörpers (20) etwa bündig schließt.

4. Ansatzstück nach einem der Ansprüche 1 bis 3, dadurch gekennzeichnet, daß der Schlauch (10) aus einem schwer verklebbaren Werkstoff, wie PE, PP, PTFE, FEP, EVA, PUR, besteht, der Ansatzstückkörper (20) ein Spritzgießteil aus anderem Kunststoff ist und der Klebstoff (27) ein Ein- und/oder Zweikomponentenkleber auf Basis von Polyester oder Epoxid oder Methacrylat oder PUR oder SIR ist.

5. Ansatzstück nach einem der Ansprüche 1 bis 3, dadurch gekennzeichnet, daß der Schlauch (10) aus einem schwer verklebbaren Werkstoff, wie PE, PP, PTFE, FEP, EVA, PUR, besteht, der Ansatzstückkörper (20) ein Spritzgießteil aus anderem Kunststoff ist und der Klebstoff (27) ein UV-härtender Klebstoff ist.

FIG.1

FIG.2

Europäisches
Patentamt

**EUROPÄISCHER
RECHERCHENBERICHT**

Nummer der Anmeldung

**EP 91 10 3355**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | EP-A-0 307 687 (SWF AUTO-ELECTRIC)<br>* Spalte 4, Zeile 29 - Zeile 34 * * Spalte 6, Zeile 23 - Zeile 34; Abbildung 1 *<br>– – – | 1-5 | A 61 M 39/00<br>F 16 L 33/00<br>B 29 C 65/54 |
| Y | US-A-4 256 333 (JONES)<br>* Zusammenfassung; Abbildungen *<br>– – – | 1-5 | |
| A | PATENT ABSTRACTS OF JAPAN vol. 9, no. 87 (M-372)(1810) 17. April 1985<br>& JP-A-59 215 817 (INOUE EMU TEE PII K.K ) 5. Dezember 1984<br>* das ganze Dokument *<br>– – – | 1-5 | |
| A | DE-B-1 158 779 (MAX WIDENMANN ARMATURENFA-BRIK)<br>* Spalte 1, Zeile 29 - Zeile 50; Abbildungen *<br>– – – | 1,2 | |
| A | DE-A-3 604 922 (METZELER KAUTSCHUK GMBH)<br>* Zusammenfassung; Abbildungen *<br>– – – | 1,2 | |
| A,D | US-A-3 851 647 (MONESTERE JR ET AL)<br>* Spalte 2, Zeile 6 - Zeile 16; Abbildung 1 *<br>– – – – – | 1-5 | RECHERCHIERTE SACHGEBIETE (Int. Cl.5)<br><br>A 61 M<br>F 16 L<br>B 29 C<br>F 16 B |

Der vorliegende Recherchenbericht wurde für alle Patentansprüche erstellt

| Recherchenort | Abschlußdatum der Recherche | Prüfer |
|---|---|---|
| Den Haag | 24 Mai 91 | CLARKSON P.M. |